# EUROPEAN PATENT APPLICATION

(11) **EP 1 495 761 A1**
(43) Date of publication of application: **12.01.2005**
(21) Application number: 03746472.4
(22) Date of filing: 14.04.2003
(51) Int. Cl.: A61K 31/729, A61P 29/00, C08B 37/12, A23L 1/30, A23L 1/337

(54) **REMEDIES**

(30) Priority: 17.04.2002 JP 2002114384; 11.11.2002 JP 2002327504
(71) Applicant: TAKARA BIO INC., Otsu-shi, Shiga 520-2193 (JP)
(72) Inventor: NISHIYAMA, Eiji, Moriyama-shi, Shiga 524-0102 (JP); MURAKI, Nobuko, Koka-gun, Shiga 529-1851 (JP); OHNOGI, Hiromu, 311, Hamoparesu Kusatsu, Kusatsu-shi, Shiga 525-0025 (JP); SAGAWA, Hiroaki, Kusatsu-shi, Shiga 525-0025 (JP); KATO, Ikunoshin, Shiga 529-1851 (JP)
(74) Representative: Schnappauf, Georg Dr.
(86) International application number: PCT/JP2003/004680
(87) International publication number: WO 2003/086422

(57) **Abstract**

Remedies or preventives, which are characterized by containing as the active ingredient at least one member selected from the group consisting of agar, agarose, high-molecular weight digestion products originating in agar and high-molecular weight digestion products originating in agarose, for diseases sensitive to the above active ingredients.

## Description

### Technical Field

The present invention relates to a pharmaceutical composition, food, drink or feed that contains a physiologically active substance derived from an alga as an active ingredient.

### Background Art

It is considered that inflammation results from protective action of a living body against invasion from outside, which action causes production of endogenous active substances to allow the physical condition to adapt. However, the response caused by the above-mentioned events often becomes harmful and causes disease states. Inflammation associated with an autoimmune disease results from recognition of autologous cells as foreign substances by immune cells, which results in damaging of normal cells.

Rheumatoid arthritis, one of autoimmune diseases, is inflammation specific to joints. As pharmacotherapies for rheumatoid arthritis, internal therapies using steroidal or non-steroidal antiinflammatory drugs and remission-inducing drugs (gold, D-penicillamine, etc.) are conducted.

Low molecular agarooligosaccharides derived from algae (e.g., agar) are expected to be developed as materials for foods, and their antirheumatic activity has been reported (e.g., WO 00/43018).

### Objects of Invention

The main object of the present invention is to develop a substance that is suitable as a material for a food or a pharmaceutical composition and highly safe, and to provide a pharmaceutical composition, food, drink or feed that contains the substance as an active ingredient.

### Summary of Invention

The present invention is outlined as follows. The first aspect of the present invention relates to a pharmaceutical composition containing, as an active ingredient, at least one selected from the group consisting of agar, agarose and a macromolecular degradation product derived from agar or agarose, said composition being used for treating or preventing a disease that is sensitive to said active ingredient.

The second aspect of the present invention relates to a food, drink or feed containing, as an active ingredient, at least one selected from the group consisting of agar, agarose and a macromolecular degradation product derived from agar or agarose, said food, drink or feed being used for treating or preventing a disease that is sensitive to said active ingredient.

According to the first or second aspect, the disease is exemplified by rheumatism or a disease that requires suppression of inflammation for its treatment or prevention. A macromolecular degradation product that is soluble in water at normal temperature, a macromolecular degradation product that results in gel with a jelly strength of 10-250 g/cm² when the gel is prepared by dissolving the macromolecular degradation product at a concentration of 1.5%(w/v), or a macromolecular degradation product that consists of eight or more constituting saccharide molecules can be used as the macromolecular degradation product.

### Brief Description of Drawings

Figure 1 illustrates the relationship between the administration of Ultra Agar and the arthritis score.
Figure 2 illustrates the relationship between the administration of Ultra Agar and the incidence rate.
Figure 3 illustrates the relationship between the administration of Ultra Agar and the arthritis score.
Figure 4 illustrates the relationship between the administration of Ultra Agar and the incidence rate.
Figure 5 illustrates the relationship between the administration of the soluble macromolecular degradation product fraction and the arthritis score.
Figure 6 illustrates the relationship between the administration of the soluble macromolecular degradation product fraction and the incidence rate.
Figure 7 illustrates the relationship between the fasting administration of Ultra Agar and the arthritis score.

### Detailed Description of the Invention

There is no specific limitation concerning the raw material used for obtaining agar and/or agarose or a macromolecular degradation product thereof according to the present invention as long as it contains agar and/or agarose. For example, one selected from red algae belonging to *Gelidiaceae* (e.g., *Gelidium amansii, Gelidium japonicum, Gelidium pacificum, Gelidium subcostatum, Pterocladia tenuis* and *Acanthopeltis japonica*), red algae belonging to *Gracilariaceae* (e.g., *Gracilaria verrucosa* and *Gracilaria gigas*), red algae belonging to *Ceramiaceae* (e.g., *Ceramium kondoi and Campylaephora hypnaeoides*), and other red algae is used as a raw material for agar or agarose. A mixture of several kinds of algae is usually used as the raw material. An alga dried in the sun is usually used as the raw material alga, although either a fresh alga or a dried alga can be used according to the present invention. An alga which has been bleached while spraying water during drying (a so-called bleached raw alga) can also be used.

The raw material alga is extracted with hot water and then cooled to obtain so-called "gelidium jelly". Agar is obtained by removing water from the "gelidium jelly" by freeze-dehydration or compress-dehydration, and then drying it. Agar in any one of various forms such as bar, belt, board, thread and powder can be used according to the present invention regardless of the source alga. Commercially available agar with varying strength can be used. Agar usually contains about 70% of agarose and about 30% of agaropectin. Agar can be further purified to prepare agarose with higher purity. Purified agarose with high purity or low purity and varying agarose content can be used.

The raw materials used according to the present invention include the above-mentioned raw material algae for agar, gelidium jelly, agar and purified agarose as well as intermediate products and byproducts obtained during preparation of these substances.

The raw materials according to the present invention also include a partially degraded product of the above-mentioned raw material which is obtained using a chemical, physical and/or enzymatic method.

The macromolecular degradation product derived from agar and/or agarose used according to the present invention (hereinafter also referred to as a macromolecular degradation product according to the present invention) can be obtained by degrading a raw material using a chemical, physical and/or enzymatic method.

Examples of chemical decomposition methods include hydrolysis under acidic to neutral conditions. Examples of physical decomposition methods include fragmentation or degradation by radiation of electromagnetic waves or ultrasonic waves. Examples of enzymatic digestion methods include hydrolysis with a hydrolase (e.g., agarase).

The acidic to neutral conditions for degrading the raw material can be determined such that the antirheumatic or antiinflammatory activity of the resulting degradation product is maximized.

The thus obtained macromolecular degradation product according to the present invention can be purified using the antirheumatic or antiinflammatory activity as an index. A known purification method such as a chemical method or a physical method may be used for the purification. The active ingredient may be enriched by using a combination of conventional purification methods such as gel filtration, fractionation using a molecular weight fractionating membrane, solvent extraction and various chromatographies using ion exchange resins or the like.

A macromolecular degradation product that is soluble in water at normal temperature exemplifies the macromolecular degradation product that can be preferably used according to the present invention. As used herein, normal temperature refers to 25°C. An aqueous solution of the macromolecular degradation product has a physical property that results in smooth (not slimy) fluidity in spite of the high molecular weight. Alternatively, a soluble fraction which is obtained, for example, by separation from an insoluble fraction by centrifugation of a prepared aqueous solution of Ultra Agar can be used as the macromolecular degradation product according to the present invention.

A macromolecular degradation product that results in gel with a jelly strength of 10-250 g/cm² when the gel is prepared by dissolving the macromolecular degradation product at a concentration of 1.5%(w/v) exemplifies the macromolecular degradation product that can be preferably used according to the present invention. The low-strength agar disclosed in Japanese Patent No. 3023244 exemplifies such a macromolecular degradation product. One prepared by subjecting agar to acid decomposition according to the method as described in the patent may be used as the macromolecular degradation product. A commercially available macromolecular degradation product can be used as the macromolecular degradation product. For example, the low-strength agar commercially available from Ina Food Industry (product name: Ultra Agar AX-30T) can be used.

A macromolecular degradation product consisting of eight or more constituting saccharide molecules exemplifies the macromolecular degradation product that can be preferably used according to the present invention. It is exemplified by a macromolecular degradation product consisting of for example 10 or more, preferably 10 to 10000, more preferably 10 to 8000, most preferably 10 to 6000 constituting saccharide molecules.

Macromolecular degradation products that are soluble in water at normal temperature and macromolecular degradation products consisting of eight or more constituting saccharide molecules used as the macromolecular degradation products according to the present invention include a macromolecular degradation product that results in gel with a jelly strength of 10 g/cm² or less when the gel is prepared by dissolving the macromolecular degradation product at a concentration of 1. 5% (w/v).

The present inventors previously found that an agarooligosaccharide has a strong antiinflammatory activity on a mouse type II collagen-induced arthritis model (WO 00/43018). Such an effect is exerted using one tenth amount of agar, agarose or a macromolecular degradation product thereof according to the present invention. One of the predicted causes is that the agarooligosaccharide administered into an animal is susceptible to degradation in vivo, whereas the macromolecular degradation product according to the present invention may be degraded in vivo and converted into a form that is more preferable for exerting the effect according to the present invention. This finding has been obtained for the first time by carrying out animal experiments as described in Examples below. Thus, agar, agarose or a macromolecular degradation product thereof according to the present invention is very useful as a material for a pharmaceutical composition, food or drink.

Agar, agarose or a macromolecular degradation product thereof according to the present invention has an antirheumatic or antiinflammatory activity. Then, a pharmaceutical composition containing, as an active ingredient, agar, agarose or a macromolecular degradation product thereof according to the present invention, for example, for treating or preventing rheumatism or a disease that requires suppression of inflammation for its treatment or prevention is provided.

Rheumatism is a disease in which synoviocytes or chondrocytes are damaged. The substance used as an active ingredient according to the present invention is useful as an antirheumatic agent due to its antiproliferation activity against synoviocyte or the like.

The substance used according to the present invention inhibits the production of tumor necrosis factor. It is considered that tumor necrosis factor directly causes inflammation in organ-specific autoimmune diseases (e.g., rheumatoid arthritis) and inflammatory diseases. Thus, it ameliorates disease states of inflammation and rheumatism (in particular, rheumatoid arthritis), resulting in marked decrease in inflammation markers including a C-reactive protein (CRP) value, a rheumatoid factor (RF) value and an erythrocyte sedimentation rate value, as well as remarkable amelioration of complications such as dysbasia.

Diseases that are sensitive to the substance used as an active ingredient according to the present invention are exemplified by diseases accompanying inflammation, rheumatic diseases and autoimmune diseases. Examples of diseases accompanying inflammation include bronchitis, pneumonia, gastritis, appendicitis, hepatitis, cholecystitis, pancreatitis, nephritis, ulcerative colitis, Crohn's disease, stomatitis, gingivitis, dermatitis, angitis and arteriosclerosis. Examples of rheumatic diseases include rheumatoid arthritis, osteoarthritis and gout. Examples of autoimmune diseases include diabetes, systemic lupus erythematosus, autoimmune hemolytic anemia, multiple sclerosis, pollinosis, allergic inflammation and organ-specific autoimmune diseases. In addition, the active ingredient according to the present invention is expected to exert various effects of preventing carcinogenesis due to its antiinflammatory activity.

The above-mentioned pharmaceutical composition for treatment or prevention of the present invention can be formulated by using, as an active ingredient, agar, agarose and/or a macromolecular degradation product thereof according to the present invention, and formulating it with a known pharmaceutical carrier.

The active ingredient is generally mixed with a pharmaceutically acceptable liquid or solid carrier and, optionally, a solvent, a dispersing agent, an emulsifier, a buffering agent, a stabilizer, an excipient, a binder, a disintegrant, a lubricant or the like to formulate it. The formulation may be in a form of a solid preparation such as tablet, granule, powder, epipastic or capsule, or a liquid preparation such as a normal solution, a suspension or an emulsion. In addition, it may be formulated into a dried preparation, which can be reconstituted as a liquid preparation by adding an appropriate carrier before use.

The pharmaceutical composition for treatment or prevention of the present invention can be administrated either as an oral preparation, or as a parenteral preparation such as an injectable preparation or drops.

The dosage of the pharmaceutical composition for treatment or prevention of the present invention is appropriately determined and varies depending on the particular dosage form, the administration route and the object as well as the age, the body weight and the conditions of the patient to be treated. In general, a daily dosage for an adult person is for example 100 µg to 1000 mg/kg, preferably 500 µg to 500 mg/kg, more preferably 1 mg to 200 mg/kg in terms of the amount of the active ingredient contained in the formulation. Of course, the dosage can vary depending on various factors. Therefore, in some cases, a less dosage than the above may be sufficient but, in other cases, a dosage more than the above may be required. The pharmaceutical composition of the present invention can be administrated orally as it is, or it can be taken daily by adding to an arbitrary food or drink.

The food, drink or feed of the present invention is a food, drink or feed which contains, which is produced by adding thereto, and/or which is produced by diluting agar, agarose and/or a macromolecular degradation product thereof according to the present invention. Due to its antirheumatic or antiinflammatory activity, it is very useful for ameliorating the disease states of or preventing rheumatism or a disease that requires suppression of inflammation for its treatment or prevention which is sensitive to agar, agarose and/or a macromolecular degradation product thereof according to the present invention.

There is no specific limitation concerning the process for producing the food, drink or feed of the present invention. Any process generally employed for producing a food, drink or feed can be used as long as the resultant food, drink or feed contains, as an active ingredient, agar, agarose and/or a macromolecular degradation product thereof according to the present invention.

There is no specific limitation concerning the food or drink of the present invention. Examples thereof include products of processed cereal, products of processed fat and oil, products of processed soybeans, processed marine products, dairy products, products of processed vegetables and fruits, confectioneries, alcohol drinks, luxury drinks, seasonings, canned, bottled or bagged foods, semi-dried or condensed foods, dried foods, frozen foods, solid foods, liquid foods, processed agricultural or forest products (e.g., spice), processed livestock products and processed marine products.

As long as the food or drink of the present invention contains, is produced by adding thereto, and/or is produced by diluting the above-mentioned active ingredient(s) in an amount necessary for exerting the antirheumatic or antiinflammatory activity, there is no specific limitation concerning its form. The food, drink or feed encompasses one in any edible form such as tablet, granule and capsule.

If a food in a form of tablet is to be produced as the food of the present invention, for example, powder of agar, agarose and/or a macromolecular degradation product thereof according to the present invention can be granulated using a known method. Examples of granulation methods include, but are not limited to, rolling granulation, stirring granulation, fluidized bed granulation, air flow granulation, extrusion granulation, compression molding granulation, shredding granulation, injection granulation and spray granulation. Other components such as cellulose, eggshell calcium, a sucrose ester of fatty acid, starch, calcium carbonate, lactose, sugar, reducing maltose or a flavoring agent may be mixed upon granulation.

There is no specific limitation concerning the content of agar, agarose or a macromolecular degradation product thereof in the food or drink of the present invention. The content can be appropriately determined in view of the sensory characteristics and exertion of the activity. For example, it is contained in the food at a concentration of 0.001 or more, preferably 0.01-100, more preferably 0.1-100% by weight. For example, 100 µg to 1000 mg/kg, preferably 500 µg to 500 mg/kg, more preferably 1 mg to 200 mg/kg is taken on a daily basis for an adult person in terms of the amount of the contained active ingredient.

The feed of the present invention is, for example, for domestic animals, farmed fishes, poultry or pets. It means an artificial food or drink for organisms other than humans which contains agar, agarose and/or a macromolecular degradation product thereof according to the present invention. The content as described above with respect to the food or drink of the present invention can be applied to the content in the feed of the present invention.

No acute toxicity was observed when agar, agarose and/or a macromolecular degradation product thereof used according to the present invention was orally or intraperitoneally administered to a mouse at a dosage of 1 g/kg.

### Examples

The following examples further illustrate the present invention in detail but are not to be construed to limit the scope thereof.

### Example 1: Therapeutic effect of Ultra Agar freely given in drinking water on mouse type II collagen-induced arthritis model

An emulsion prepared by mixing 3 mg/ml of type II collagen derived from bovine joint (K41: Collagen Gijutsu Kenshukai) and an equal volume of Freund's complete adjuvant was subcutaneously administered (150 µg/mouse) at a ridge portion of a mouse (DBA/1J, male, 6 weeks old, purchased from Seac Yoshitomi). After 3 weeks, the mouse was boosted in a similar manner to induce type II collagen-induced arthritis. Each group consisted of ten mice.

A dilution of Ultra Agar (AX-30T, Ina Food Industry) at a concentration of 0.3% in tap water was freely given in drinking water after boosting with collagen in order to examine its therapeutic effect. A housing cage from Clea Japan (product name: Econ PC, CL-0106-1) and a watering bottle from Clea Japan (product name: Watering Bottle Set CK-200, CL-0902/ with K-1) were used. It was observed that a part of Ultra Agar remained insoluble and precipitated in the watering bottle upon the administration.

Preparation of the Ultra Agar solution and exchange of the watering bottle were carried out three times a week (Monday, Wednesday and Friday). Tap water was given to a control group in a similar manner.

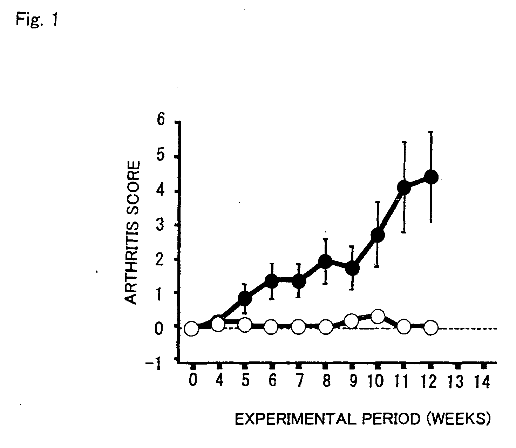

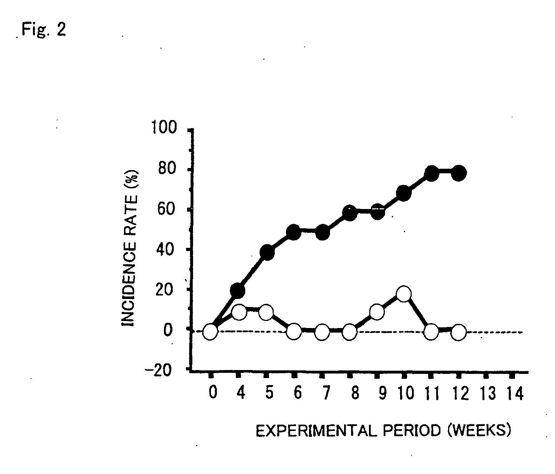

The onset of arthritis was assessed by scoring for each limb as follows. An arthritis score for a mouse was expressed as a sum of scores for four limbs (the highest score: 16). 0: no change; 1: swelling in one or plural limb(s); 2: rubefaction and swelling observed overall; 3: severe swelling observed overall; and 4: accompanying tonic change in joint. In addition, the incidence rate was also calculated. The results are shown in Figures 1 and 2. Figure 1 illustrates the relationship between the administration of Ultra Agar and the arthritis score. The longitudinal axis represents the arthritis score (mean value for ten mice) and the horizontal axis represents the experimental period (weeks). In the figure, the closed circles represent values for the control group, and the open circles represent values for the group with the administration of Ultra Agar. Figure 2 illustrates the relationship between the administration of Ultra Agar and the incidence rate. The longitudinal axis represents the incident rate (%, for ten mice) and the horizontal axis represents the experimental period (weeks).

As a result, suppression of the arthritis score and an almost complete therapeutic effect were observed with the intake of Ultra Agar (AX-30T) in drinking water at a concentration of 0.3% (about 0.6 g/kg body weight/day) as shown in Figures 1 and 2. No significant difference in the amount of taken water, the amount of taken feed or change in the body weight was observed between the control group and the administration group during the experimental period.

### Example 2: Therapeutic effect of forced oral administration of Ultra Agar on mouse type II collagen-induced arthritis model

Type II collagen-induced arthritis was induced in mice as described in Example 1. Each group consisted of ten mice.

A diluted suspension of Ultra Agar (AX-30T, Ina Food Industry) at a concentration of 1% in distilled water was used for forced oral administration at a dosage of 100 mg/10 ml/kg given for five successive days per week after boosting with collagen.

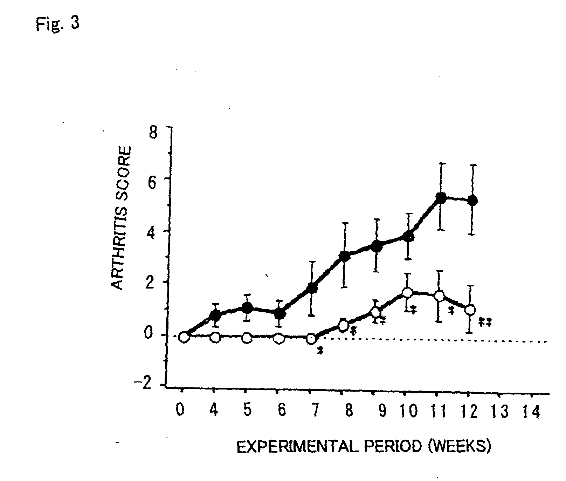

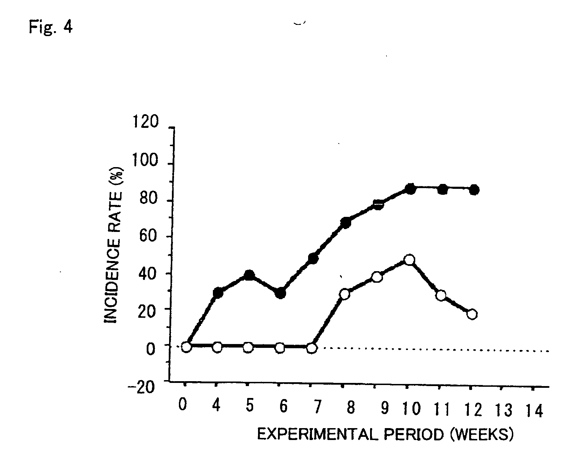

The onset of arthritis was assessed as described in Example 1. In addition, the incidence rate was also calculated. The results are shown in Figures 3 and 4. Figure 3 illustrates the relationship between the administration of Ultra Agar and the arthritis score. The longitudinal axis represents the arthritis score (mean value for ten mice) and the horizontal axis represents the experimental period (weeks). In the figure, the closed circles represent values for the control group, and the open circles represent values for the group with the administration of Ultra Agar. Figure 4 illustrates the relationship between the administration of Ultra Agar and the incidence rate. The longitudinal axis represents the incident rate (for ten mice) and the horizontal axis represents the experimental period (weeks). In the figure, the closed circles represent values for the control group, and the open circles represent values for the group with the administration of Ultra Agar.

As a result, significant improvement in the arthritis score was observed with the oral administration of Ultra Agar as shown in Figures 3 and 4. Prominent improvement in the incidence rate was also observed. No significant difference in the amount of taken water, the amount of taken feed or change in the body weight was observed between the control group and the administration group during the experimental period. Mann-Whitney U test was used to test the significance. The marks * and ** in the figures indicate significance of p < 0.05 and p < 0.01, respectively.

### Example 3: Preparation of soluble macromolecular degradation product fraction derived from Ultra Agar

200 g of Ultra Agar was added to 20 L of 10 mM citric acid aqueous solution. The mixture was heated at 95°C for 60 minutes. After cooling to room temperature, the mixture was concentrated to a volume of 2 L using a rotary evaporator. 3 L of ethanol was added thereto. The mixture was stirred and then centrifuged at 5000 g for 10 minutes to obtain a Precipitate 1 and a Supernatant 1. The thus obtained Precipitate 1 was dissolved in distilled water (final volume of 1.1 L), and the solution was dialyzed against distilled water using a dialysis membrane Spectra/Por 7 Membrane MWCO 1,000 (Spectrum Medical Industries). The dialysate was incubated at 60°C for 5 minutes, allowed to stand at room temperature to cool to 40°C, and centrifuged at 5000g for 10 minutes to obtain a Precipitate 2 and a Supernatant 2. 200 ml of distilled water was added to the thus obtained Precipitate 2. The mixture was centrifuged at 5000g for 10 minutes to obtain a Precipitate 3 and a Supernatant 3. The thus obtained Supernatant 3 and the Supernatant 2 were mixed together and lyophilized to obtain 54 g of a soluble macromolecular degradation product fraction derived from Ultra Agar.

### Example 4: Therapeutic effect of soluble macromolecular degradation product derived from Ultra Agar freely given in drinking water on mouse type II collagen-induced arthritis model

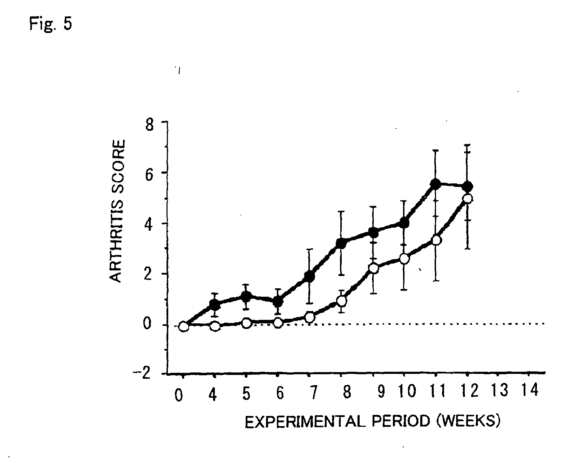

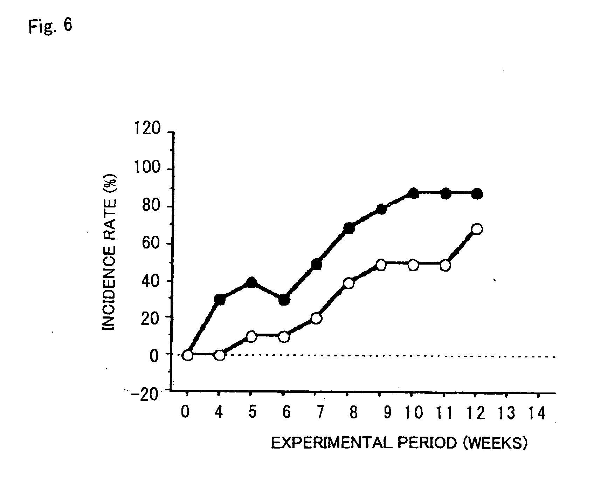

Type II collagen-induced arthritis was induced in mice as described in Example 1. Each group consisted of ten mice. A dilution of the soluble macromolecular degradation product fraction at a concentration of 0.3% in tap water was freely given in drinking water after boosting with collagen to examine its therapeutic effect. Preparation of the dilution and exchange of the watering bottle were carried out three times a week (Monday, Wednesday and Friday). Tap water was given to a control group in a similar manner. The onset of arthritis was assessed as described in Example 1. In addition, the incidence rate was also calculated. The results are shown in Figures 5 and 6. Figure 5 illustrates the relationship between the administration of the soluble macromolecular degradation product fraction and the arthritis score. The longitudinal axis represents the arthritis score (mean value for ten mice) and the horizontal axis represents the experimental period (weeks). In the figure, the closed circles represent values for the control group, and the open circles represent values for the group with the administration of the soluble macromolecular degradation product fraction. Figure 6 illustrates the relationship between the administration of the soluble macromolecular degradation product fraction and the incidence rate. The longitudinal axis represents the incident rate (for ten mice) and the horizontal axis represents the experimental period (weeks). In the figure, the closed circles represent values for the control group, and the open circles represent values for the group with the administration of the soluble macromolecular degradation product fraction.

As a result, improvement in the arthritis score was observed with the intake of the soluble macromolecular degradation product fraction in drinking water as shown in Figures 5 and 6. Prominent improvement in the incidence rate was also observed. Thus, effects of suppressing the arthritis score and the incidence rate were observed with the intake of the soluble macromolecular degradation product fraction in drinking water. No significant difference in the amount of taken water, the amount of taken feed or change in the body weight was observed between the control group and the administration group during the experimental period.

### Example 5: Therapeutic effect of fasting administration of Ultra Agar on mouse type II collagen-induced arthritis model

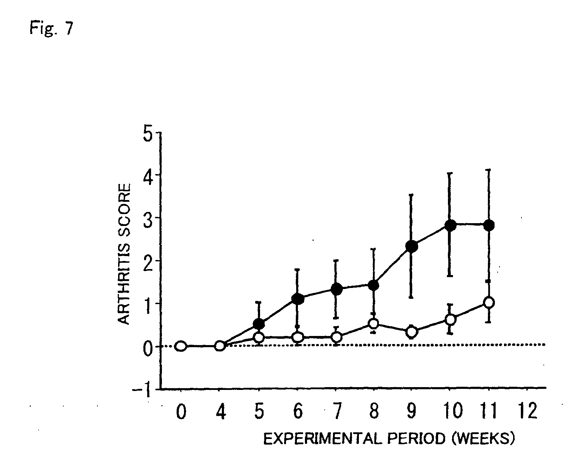

Type II collagen-induced arthritis was induced in mice as described in Example 1. Each group consisted of ten mice. A diluted suspension of Ultra Agar (AX-30T, Ina Food Industry) at a concentration of 1% in distilled water was used for fasting administration at a dosage of 100 mg/10 ml/kg after boosting with collagen. Specifically, the mice were starved for feed and water from 9 a.m., and subjected to forced oral administration of Ultra Agar prepared as described above at 3 p.m. The starvation for feed and water was further maintained, and feeding and drinking were resumed at 5 p.m. Distilled water was given to a control group in a similar manner. The fasting administration was carried out for five successive days per week. The results are shown in Figure 7. Figure 7 illustrates the relationship between the fasting administration of Ultra Agar and the arthritis score. The longitudinal axis represents the arthritis score (mean value for ten mice) and the horizontal axis represents the experimental period (weeks). In the figure, the closed circles represent values for the control group, and the open circles represent values for the group with the fasting administration of Ultra Agar.

As a result, prominent improvement in the arthritis score was observed with the fasting administration of Ultra Agar as shown in Figure 7.

### Example 6: Preparation of tableted food

Three types of tablets (200 mg/tablet) each containing agar as an active ingredient and having the compositions as shown in Tables 1 to 3 were prepared according to a conventional method using a tableting machine with tablet compression pressure of 3000 kg/cm².

**Table 1:**

| Composition (per tablet) | | |
|---|---|---|
| Ultra Agar | (mg) | 160 |
| Crystalline cellulose | (mg) | 16 |
| Eggshell calcium | (mg) | 16 |
| Sucrose ester of fatty acid | (mg) | 8 |
| Total | | 200 |

**Table 2:**

| Composition (per tablet) | | |
|---|---|---|
| Ultra Agar | (mg) | 116 |
| Crystalline cellulose | (mg) | 15 |
| Reducing maltose | (mg) | 30 |
| Starch | (mg) | 30 |
| Sucrose ester of fatty acid | (mg) | 8 |
| Calcium carbonate | (mg) | 1 |
| Total | | 200 |

**Table 3:**

| Composition (per tablet) | | |
|---|---|---|
| Ultra Agar | (mg) | 122 |
| Crystalline cellulose | (mg) | 10 |
| Lactose | (mg) | 45 |
| Reducing maltose | (mg) | 10 |
| Starch | (mg) | 1 |
| Sucrose ester of fatty acid | (mg) | 8 |
| Flavoring agent (peppermint) | (mg) | 4 |
| Total | | 200 |

### Industrial Applicability

The present invention provides an antirheumatic pharmaceutical composition which has an activity higher than conventional ones. The pharmaceutical composition is particularly useful because the activity-exerting period is long. The present invention also provides a highly functional food, drink or feed. It is very useful as an antiinflammatory food, drink or feed for the maintenance of homeostasis in a living body.

## Claims

1. A pharmaceutical composition containing, as an active ingredient, at least one selected from the group consisting of agar, agarose and a macromolecular degradation product derived from agar or agarose, said composition being used for treating or preventing a disease that is sensitive to said active ingredient.

2. The composition according to claim 1, wherein the disease is rheumatism or a disease that requires suppression of inflammation for its treatment or prevention.

3. The composition according to claim 1 or 2, wherein the macromolecular degradation product is soluble in water at normal temperature.

4. The composition according to claim 1 or 2, the jelly strength of gel prepared by dissolving the macromolecular degradation product at a concentration of 1.5%(w/v) is 10-250 g/cm².

5. The composition according to claim 1 or 2, wherein the macromolecular degradation product consists of eight or more constituting saccharide molecules.

6. A food, drink or feed containing, as an active ingredient, at least one selected from the group consisting of agar, agarose and a macromolecular degradation product derived from agar or agarose, said food, drink or feed being used for treating or preventing a disease that is sensitive to said active ingredient.

7. The food, drink or feed according to claim 6, wherein the disease is rheumatism or a disease that requires suppression of inflammation for its treatment or prevention.

8. The food, drink or feed according to claim 6 or 7, wherein the macromolecular degradation product is soluble in water at normal temperature.

9. The food, drink or feed according to claim 6 or 7, the jelly strength of gel prepared by dissolving the macromolecular degradation product at a concentration of 1.5%(w/v) is 10-250 g/cm².

10. The food, drink or feed according to claim 6 or 7, wherein the macromolecular degradation product consists of eight or more constituting saccharide molecules.
